# EUROPEAN PATENT SPECIFICATION

(11) **EP 1 231 929 B1**
(45) Date of publication and mention of the grant of the patent: **20.07.2005**
(21) Application number: 00914291.0
(22) Date of filing: 30.03.2000
(51) Int. Cl.: A61K 35/80, A23L 1/337, A61P 31/12, A61P 31/22

(54) **ANTIVIRAL COMPOSITION COMPRISING A PALMARIA PALMATA EXTRACT**
ANTI-VIRALE ZUSAMMENSETZUNG ENTHALTEND EIN PALMARIA PALMATA EXTRAKT
COMPOSITION ANTIVIRALE COMPRENANT UN EXTRAIT DE PALMARIA PALMATA

(30) Priority: 01.04.1999 GB 9907596
(43) Date of publication of application: 21.08.2002
(73) Proprietor: Henderson Morley Limited, Birmingham B13 8JS (GB)
(72) Inventor: PARDOE, Ian Stuart, Henderson Morley Limited, Birmingham B13 8JS (GB)
(74) Representative: Symons, Rupert Jonathan
(86) International application number: PCT/GB2000/001233
(87) International publication number: WO 2000/059524

(56) References cited:
- WO-A-91/07946

## Description

The invention relates to an anti-viral composition useful in the therapeutic or prophylatic treatment of a range of viral conditions. More particularly the invention is useful in the treatment of Herpes family viral infections.

WO 91.07946 discloses the inclusion of extracts of algae in the preparation of compositions having an anti-radical activity. In Example 6 the document discloses the extraction of an extract for this purpose from Palmana palmata using water and isopropanol.

It has been suggested that certain marine algal species have anti-viral activity, for example, Ehresmann, 1977, J Phycol. 13:37-40. However, the species so far identified as having anti-viral action are both rare in their occurrence and difficult to harvest as well as being impossible to grow in culture. This has limited their usefulness.

The invention is based on the realisation that extracts of Palmaria palmata are useful for the purpose specified. Palmaria palmata is different in that it is abundant, may be artificially cultured and therefore may be used more easily to mass produce an anti-viral material.

In one aspect the invention provides use of a water-soluble extract of Palmaria palmata for the production of a pharmaceutical composition effective against viruses.

In one specific aspect the invention provides use of a water soluble extract of Palmaria palmata adapted for administration in the treatment of infections caused by viruses of the Herpes family.

The composition may be formulated for application in any suitable way, e.g. as a capsule, a tablet, suppository, injectable solution, topical cream; or the like. In addition the extract may be used as an additive for a foodstuff, in a face mask; or the like.

For oral application there is provided a 200 mg. capsule, to be ingested three times daily.

The water-soluble extract of Palmaria palmata may be obtained by a method comprising drying the Palmaria palmata to a predetermined water content, comminuting the material to a fine powder and then dissolving the powder in water. The method preferably includes the step of storing the powder at or below room temperature. Preferably the method includes the step of centrifuging the solution and recovering the supernatant. Preferably the dried material is mixed with water in the ratio of 50g:300ml.

The full botanical name of Palmaria palmata is Algae Rhodophycota Rhodophyceae Palmariales Palmariacceae. Common names include Dulsk, Dillisk, Soel, Darusu, Goemon. A review of the chemical constituents of the red alga Palmaria palmata is published in Economic Botany 34(1), 1980, pp 27-50. This discloses at page 36 that the major polysaccharide is a water-soluble xylan composed of β - (1 → 3) and β - (1 → 4) linked D-xylose units which contain no sulphate ester or methoxyl groups.

The water soluble extracts of Palmaria palmata are extremely effective for the treatment of viruses of the Herpes family including Herpes simplex type 1, Herpes simplex type 2, varicella zoster cytomegalovirus, human Herpes type 6 and human Herpes type 8. The anti-viral activity of these water soluble extracts may also be effective against other viruses.

Biochemical analyses of Palmaria palmata show that the major components are as follows:
**Protein:**
   The classical Kjeldahl method support a high protein content of Palmaria palmata.
**Lipids:**
   Lipids may be extracted using a solvent into which lipids dissolve, e.g. chloroform.
   Minor components include vitamins and minerals including carotene, ascorbic acid, calcium, iron, and iodine.
**Carbohydrates:**
   The full botanical name of Palmaria palmata is Algae Rhodophycota Rhodophyceae Palmariales Palmariacceae. Common names include Dulsk, Dillisk, Soel, Darusu, Goemon. A review of the chemical constituents of the red alga Palmaria palmata is published in Economic Botany 34(1), 1980, pp 27-50. This discloses at page 36 that the major polysaccharide is a water-soluble xylan composed of β - (1 → 3) and β - (1 → 4) linked D-xylose units which contain no sulphate ester or methoxyl groups. The carbohydrate fraction of Palmaria palmata contains easily soluble polysaccharides and more fibre like polymers which act similarly to cellulose. It is possible to fractionate the carbohydrates found in Palmaria palmata by extraction/precipitation.

While we do not wish the invention to be limited by the following theory our evaluations suggest that as the carbohydrate fraction is the only major water soluble component of the algae, the anti-viral action is from a complex carbohydrate and most probably polysaccharide in nature. The present knowledge of complex carbohydrates does not enable the large scale production or manufacture of these very complex molecules. For this reason it is believed that for a pharmaceutical compound to be successfully derived from Palmaria palmata, it will be extracted from cultured plants.

A sample of Palmaria palmata (Linnaeus Kuntze) belonging to the family Rhodophyta (Common name Dulse, Dillisk, Dilleasc, Darusa, Soel, Goemon a vache Creathnach) was harvested from the sea and identified as Palmaria palmata by the National University of Ireland in Galway, Department of Marine Biology, Ireland, using molecular biology techniques to specifically identify the genome of Palmaria palmata. This was confirmed by the physical appearance of the specimen conforming to that expected. This seaweed has a reddish brown colour with membranous or leathery flattened fronds 50 - 300 mm long arising from a discoid base usually with a small stipe expanding to form a simple or dichotomously and palmately divided fronds often with characteristic marginal leaflets. This seaweed may be found in many oceans attached to rock, mussels in intertidal or shallow subtidal waters, it is widely distributed and abundant.

Palmaria palmata may be cultivated for harvesting in any suitable way. We envisage that portions of the plant will be attached to ropes or tubes in sites having strong tidal currents but which are sheltered from excessive wave action. An alternative way may be to cultivate the plants in tanks having controlled concentration of essential nutrients and possibly using heated waste water.

Once harvested, the sample of Palmaria palmata was subsequently dried at 3 temperatures, namely 4°C, 25°C and 65°C, to a water content of approximately 10%. Once dried the algae may then be comminuted by any suitable method (use of pestle and mortar or liquidiser) to a fine powder. This powder may then be stored at room temperature or indefinitely at - 20°C prior to use. The powder is mixed with sterile purified water and shaken periodically either manually or by a proprietary shaker and subsequently left overnight in a refrigerator. The resultant mixture is then centrifuged at 3000 rpm for 30 minutes and the supernatant is kept for further analysis and experimentation.

It is preferred that the dried algal powder be present in the proportions of the order of 50 g Palmaria palmata and 300 mls water. The aqueous extract is preferably sterilised by passage through a proprietary millipore or other bacterial filter.

### The Herpes Simplex Virus

The herpes simplex virus (HSV) is a large (150 - 200nnm) DNA virus which consists of approximately 152,000 base pairs of double stranded DNA encapsulated in a proteinaceous capsid. The capsid is surrounded by a less well defined structure known as the tegument. The virus is contained in a host cell derived lipid bilayer which is studded with virus specific glycoproteins and integral membrane proteins.

### Genital Herpes

Recurrent herpes genitalis is estimated to affect over 45 million people in the USA alone. (Fleming et al NEJM Oct 97). The incidence is increasing, leading to a massive reservoir for transmission of infection to sexual partners and new-bom infants. Once infected, over 95% of patients who have proven primary herpes genitalis have had at least one recurrence with an average of 5 - 6 recurrences per year. Approximately 25% of people will have recurrences at monthly intervals accompanied by extremely troublesome physical discomfort and (often) psychological upset. Current therapy for genital herpes revolves around psychological support, education, barrier methods of contraception and treatment on a regular basis with the expensive anti-viral drugs such as Aciclovir, Penciclovir and Famciclovir. These are not tolerated by all sufferers and in some cases are ineffective.

### Genital Herpes in Pregnancy

Herpes simplex infection may be spread from mother to foetus. This usually occurs during parturition but can rarely occur in-utero. The rate of transmission depends on whether the mother is suffering primary herpes, where the transmission rate is 50% or recurrent herpes where the transmission rate is 8%.

If any lesions are visible during the onset of labour, this would be an indication for undertaking a caesarian section.

### Neonatal Infection

50 - 60% of infants with neonatal Herpes simplex virus infection are born to mothers with no history of genital herpes infection. Neonatal herpes simplex virus infection is very serious. Over 70% of cases present infections localised to the eyes, skin or mouth within 3 - 30 days of birth. Unfortunately, over three quarters of those infected will progress to disseminated or CNS involvement which carries a very high morbidity and mortality.

### Herpes Encephalitis

Acute encephalitis caused by herpes simplex virus is a common and often fatal infection. The herpes viruses have a role in other pathology.

### (i). Neurological Diseases: Bells Palsy

There is now compelling evidence to suggest that Bells palsy is caused by an acute herpes simplex infection of the facial nerve. In a study by Murakami (Annals of Internal Medicine 124 pt 1 27 - 30, 1996 Jan) PCR of endoneural fluid revealed HSV genomes in 11/14 patients while in healthy controls and those with Ramsay Hunt syndrome the virus could not be detected. A recently published placebo controlled double blind trial treating those with Bells palsy with either prednisolone alone or prednisolone with acyclovir showed a very statistically superior outcome when using acyclovir leading the researchers to suggest that bells palsy is probably caused by herpes simplex. (Adour et al Annals of Otology Rhinology and Laryngology 105 (5) 371 - 8 May 1996).

### (ii). Senile Dementia and Organic Brain Disease

As early as 1975 a link between herpes simplex and senile dementia was suggested. (Libikova Acta Virologica 19(6) : 493 - 5, 1975). In this study, 47% of patients suffering with senile dementia had neutralising antibodies to HSV 1 in their cerabrospinal fluid, compared to none of mentally retarded adolescents. Serum levels of antibodies were elevated in 61% of demented patients compared with 31% of normal controls. More recent work (Jamieson et al Journal of Pathology 167(4) : 365 - 8 Aug 1991) used PCR (polymerase chain reaction) to detect thymidine kinase from viral DNA. There was a significant anatomically specific presence of herpes simplex in the brains of those with senile dementia with virus being commonly detected in the hippocampus and the cortex but unversally absent in the occipital cortex.

### (iii). Inflammatory Bowel Disease

An interesting study by Wakefield (Journal of Medical Virology 1992 Vol. 38 3 : 183 - 190) demonstrated herpes virus DNA in the large intestine of biopsy specimens from patients with ulcerative colitis and Crohns disease. It appeared that the presence of at least two herpes viruses were needed to cause pathology.

### (iv). Malignancy

A study by Ruther (Tumordiagnostik & Therpaie 1994 Vol. 15 No 14, pp 121 - 127) examined biopsy specimens from patients with bronchial carcinoma, pleural mesothelioma, sarcoid and asthma. He found a significant proportion were infected with several herpes viruses (HSV 1 and 2, HHV 6 and EBV) compared to no detectable virus in healthy controls. He suggested that because viruses of the herpes group can play a part in malignant transformation of cells, the role of these viruses as causative agents of malignant diseases in man should be investigated further.

### (v). Lymphoproliferative Disorders

It has been known for many years that the herpes virus Epstien Barr is the causative agent in some forms of lymphoma. Further work examining a rapidly fatal lymphoproliferative disorder I bone marrow transplant recipient (Brion et al Nouvelle revue Francaise hematologie 1995 37 6 pp 289 - 296) showed that herpes virus genome was present in tumoral cells of all analysed specimens including herpes simlex type 1.

All of the above suggest that the role of Herpes simplex virus is not only extremely widespread within the general population but its pathological effects can be just as diverse highlighting the urgent need for effective treatments. It is considered that this invention will provide such effective treatments.

In order that the invention may be well understood it will now be described by way of illustration only with reference to the accompanying examples.

### EXAMPLE 1

50 g of Palmaria palmata was dried at 65°C in air and the resultant dried plant material was comminuted in a domestic blender. The comminuted plant matter was then mixed with 300 ml of water at 19°C which had been purified by passage through a Bobby Sterilising Double Distill water purifier. This was then stirred by manual agitation and left overnight at 4°C. The resultant mixture was centrifuged at 3000 rpm for 30 minutes in a proprietary centrifuge. The supernatant was then removed and passed through a millipore bacterial filter to remove any particulate or baterial contamination. The resultant clear, pink/purple coloured liquid contains the active substance. The liquid was removed from the active compound by freeze drying using a proprietary freeze drier (e.g.) Edwards Modulyo EF4). The resultant powder has a pinkish colour. This was then encapsulated in a standard gelatin capsule at a concentration of 200 mg per capsule. This encapsulated Palmaria palmata extract was then administered to a patient as described below.

### Human Subject

A male human diagnosed as having recurrent type 2 genital herpes by culture of virus from the penile lesion by polymerase chain reaction showing type 2 herpes simplex genome, and the presence of type 2 specific IgG against herpes simplex gG2 confirms that this was recurrent rather than primary genital herpes infection. The subject was prone to frequent (usually monthly) and severe recurrences of genital herpes. The subject has suffered from these for the previous 12 years and had tried several anti-viral drugs which had proven either unhelpful or unsuitable. The outbreaks would always cause a vesicular penile eruption with typically 12 blisters on the shaft of the penis. These so-called prodromal symptoms would inevitably yield a full attack. Within 48 hours of developing the lesions, glands would swell in the groin and systemic symptoms of viraemia would develop (e.g. raised body temperature, achy joints and tiredness). The lesions on the penis would heal without scarring after approximately 14 days.

On three separate outbreaks this subject was administered by ingestion the capsules containing the freeze dried extract of Palmaria Palmata at a dose of one 200 mg capsule, three times daily for 5 days, taken as soon as symptoms of the prodrome developed. The subject described that on each occasion of the 3 outbreaks where he administered the extract during the prodromal phase, symptoms improved within 2 hours. On two occasions the outbreak was aborted i.e. the tingling of the penis did not produce the expected vesicular rash. There was no associated swelling of glands in the groin. On the occasion that the outbreak was not aborted, the attack was much less severe than normal. Only 6 lesions were evident, no swelling of groin glands was noted and no systemic symptoms were reported. Time to full healing was 6 days.

### EXAMPLE II

Bioassays with herpes simplex virus in vitro were undertaken to follow the anti-viral activity of the water extract of Palmaria palmata. Culture and assay methods follow those described in Lennette and Schmidt (1979) for herpes simplex virus and Vero cells with minor modifications.

### Herpes simplex strains used:

Type 1 herpes simplex strain HFEM is a derivative of the Rockerfeller strain HF (Wildy 1955) and Type 2 herpes simplex strain 3345 a penile isolate (Skinner et al 1977) were used as prototype strains. These prototypes were stored at high multiplicities of infection at - 80°C until needed.

### Cell Cultures

African Green Monkey kidney cells (Vero) were obtained from the national Institute of Biological standards and Control UK and were used as the only cell line for all experiments in this Example.

### Culture Media

Cells and viruses were maintained on Glasgows modified medium supplemented with 10% foetal bovine serum.

50g Palmaria palmata were dried at 65°C, comminuted in a domestic blender, mixed with 300 mls water and left overnight at 4°C and centrifuged at 3000 rpm for 30 minutes. The supernatant was removed and following sterilisation via a millipore filter subsequently diluted 1 in 5 with purified water (the stock solution).

The effects of this stock solution on Vero cells in culture was examined, as were the effects of Vero cells which had been infected with type 1 and type 2 virus at varying levels of infectivity. The effect of the stock solution on extracellular virus was also examined.

### Results

The stock solution was found to show only minor inhibition of Vero attachment and growth and showed moderate cytopathogenesis as adjudged by the morphology of cell culture. This suggests that the water soluble extract of Palmaria palmata has only minimal cytotoxic effects.

The effects of the stock solution on different multiplicities of infection (i.e. concentration of virus) may be summarised as below:

| Inhibition of hsv2 cytopathic effect. | |
|---|---|
| Multiplicity of infection (Dose of virus) | Effect of Stock Solution |
| High | ++++ |
| Medium | ++++ |
| Low | ++++ |
| ++++ signifies inhibition of virus cytopathic effect i.e. the action of the virus on destroying the vero cells was prevented. | |

This example demonstrates that virus activity was prevented by applying a small volume of stock solution to Vero cells infected with virus. Other examples have shown that at lower concentration of stock solution e.g. diluted 1 in 25, 1 in 50, 1 in 100, this anti-viral activity disappears in a concentration dependent manner. Other examples examined the effect on Herpes Simplex type 1 and other herpes viruses with almost identical results.

### EXAMPLE III

Further experiments were conducted to examine effects on extracellular virus. There was no direct virucidal activity on extracellular virus. This result supports the claim that a water soluble extract from Palmaria palmata contains anti-viral activity.

## Claims

1. Use of a water-soluble extract of Palmaria palmata for the production of a pharmaceutical composition effective against viruses.

2. Use according to Claim 1, wherein the composition is adapted for administration orally, intravenously, or topically.

3. Use according to Claim 2, adapted for ingestion orally.

4. Use according to Claim 2 or 3, comprising a 200 mg. capsule, to be ingested three times daily.

5. Use according to any preceding Claim, adapted for administration in the treatment of viral infections caused by viruses of the Herpes family.

## Patentansprüche

1. Verwendung eines wasserlöslichen Extrakts von Palmaria palmata zur Herstellung einer pharmazeutischen Zusammensetzung, die gegen Viren wirksam ist.

2. Verwendung nach Anspruch 1, wobei die Zusammensetzung für die orale, intravenöse oder topische Verabreichung angepasst ist.

3. Verwendung nach Anspruch 2, für die orale Einnahme angepasst.

4. Verwendung nach Anspruch 2 oder 3, umfassend eine Kapsel von 200 mg, die dreimal täglich einzunehmen ist.

5. Verwendung nach irgendeinem der vorhergehenden Ansprüche, angepasst für die Verabreichung bei der Behandlung von Virusinfektionen, welche durch Viren der Herpes-Familie verursacht werden.

## Revendications

1. Utilisation d'un extrait soluble dans l'eau de Palmaria palmata pour la production d'une composition pharmaceutique efficace contre des virus.

2. Utilisation selon la revendication 1, dans laquelle la composition est adaptée pour une administration par voie orale, intraveineuse ou topique.

3. Utilisation selon la revendication 2, adaptée pour une ingestion par voie orale.

4. Utilisation selon la revendication 2 ou 3, comprenant une capsule de 200 mg devant être ingérée trois fois par jour.

5. Utilisation selon l'une quelconque des revendications précédentes, adaptée pour une administration dans le traitement d'infections virales provoquées par des virus de la famille de l'herpès.
